# EUROPEAN PATENT SPECIFICATION

(11) **EP 3 970 656 B1**
(45) Date of publication and mention of the grant of the patent: **20.03.2024**
(21) Application number: 20810752.4
(22) Date of filing: 12.02.2020
(51) Int. Cl.: A61C 8/00, A61C 3/00, A61C 1/14, A61B 17/88

(54) **DENTAL FIXTURE SURGICAL CONNECTOR**
CHIRURGISCHER VERBINDER FÜR DENTALVORRICHTUNG
CONNECTEUR CHIRURGICAL POUR APPAREIL DENTAIRE

(30) Priority: 17.05.2019 KR 20190002005 U
(43) Date of publication of application: 23.03.2022
(73) Proprietor: Megagen Implant Co., Ltd., Dalseong-gun, Daegu 42921 (KR)
(72) Inventor: PARK, Kwang Bum, Daegu 42016 (KR)
(74) Representative: Patentanwälte Hemmer Lindfeld Frese Partnerschaft mbB
(86) International application number: PCT/KR2020/001990
(87) International publication number: WO 2020/235777

(56) References cited:
- KR-A- 20140 062 212
- KR-A- 20180 049 620
- KR-B1- 101 442 806
- KR-B1- 101 442 806
- KR-Y1- 200 428 099
- US-A1- 2002 025 505
- US-A1- 2003 224 327
- US-A1- 2007 037 121
- US-A1- 2009 253 098
- US-A1- 2010 167 240
- US-B1- 6 626 911

## Description

### TECHNICAL FIELD

The inventive concept relates to a surgical connector for a dental fixture, and more particularly, to a surgical connector for a dental fixture that is capable of placing a fixture in an alveolar bone by easily picking up the fixture from a fixture storing ample where the fixture is stored.

### BACKGROUND ART

An implant originally means a substitute used to restore lost human tissues. In a dental field, however, the implant refers to a series of procedures to transplant an artificial tooth.

To replace a lost dental root, the implant is a series of dental procedures to restore the function of a tooth by placing a fixture, which is a dental root formed of titanium and having no rejection to a human body, in an alveolar bone where a tooth is lost, and then fixing an artificial tooth thereto.

In the case of a general prosthesis or denture, surrounding teeth or bones may be damaged as time passes. In contrast, since the implant does not harm the surrounding tooth tissues, provides the same function and shape as a natural tooth, and has no tooth decay, the implant may be used semi-permanently.

In an artificial tooth surgery (referred to as an implant or an implant surgery), a implantation hole is formed by using a drill, which varies according to the type of a fixture, the fixture is placed in the alveolar bone and has osseointegration with the bone, and an abutment is coupled to the fixture and then crowned with a final prosthesis.

The dental implant may facilitate restoration of a single missing tooth, enhance the function of a denture for partially toothless and fully toothless patients, improve an aesthetic aspect of dental prosthesis restoration, and furthermore distribute excessive stress applied to surrounding support bone tissues and stabilize a row of teeth.

The dental implant may generally include a fixture placed as an artificial tooth root, an abutment coupled to the fixture, an abutment screw to fix the abutment to the fixture, and an artificial tooth coupled to the abutment. Before the abutment is coupled to the fixture, that is, during a period of osseointegration of the fixture to the alveolar bone, the abutment is coupled to the fixture and a coupling state is maintained.

The fixture, which is one of components of a dental implant, is a portion placed in a drill hole formed in the alveolar bone by using a drill and functions as an artificial tooth root. A screw portion is spirally provided on the outer circumference of the fixture for the firm fixing of the fixture and the alveolar bone.

Furthermore, an abutment insertion hole in which the abutment is inserted is provided in an upper end portion of the fixture by being received in the lengthwise direction thereof.

The inner wall of the abutment insertion hole has a shape matching to the outer wall of abutment and has a polygonal shape to prevent a relative rotation with respect to the fixture of the abutment.

Meanwhile, the fixture is stored by being kept in a fixture storing ample. During an implant operation, a dentist picks up a fixture stored in a storing ample by using a dental fixture operation connector.

A dental fixture operation connector according to the related art has a head portion having an outer wall in a polygonal shape. In the dental fixture operation connector according to the related art, as the head portion having a polygonal shape is inserted into the abutment insertion hole of the fixture, the fixture is picked out of the ample through the insertion coupling between the head portion and the abutment insertion hole.

However, according to the related art, as the outer wall of the head portion of the dental fixture operation connector and the inner wall of the insertion hole each have the polygonal shape as described above, when the head portion is inserted into the insertion hole, if the direction is not correct, the head portion is not easily inserted into the insertion hole, and thus the picking-up of a fixture becomes inconvenient.

US 2009/0253098 A1 describes an implant-driver assembly and an associated method. US 200//0037121 A1 describes a carry and drive device and method for a dental implant or a dental implant component with a driving recess. US 2002/0025505 A1 describes an implant delivery system including a carrier that is attachable to an implant, with a polygonal portion and a circumferential groove at the front end of the polygonal portion. KR 101 442 806 B1 shows also an implant driving tool.

### DETAILED DESCRIPTION OF THE INVENTIVE CONCEPT

### TECHNICAL PROBLEM

The inventive concept provides a dental fixture operation connector by which a fixture stored in a fixture storing ample may be quickly and conveniently picked up from the ample to be placed in an alveolar bone.

### SOLUTION TO PROBLEM

The object of the present invention is defined in claim 1. The dental fixture operation connector includes a connector body portion, a connector head portion connected to the connector body portion and is configured to be inserted into an insertion hole formed in an upper end area of a fixture, and an entrance guide portion provided on the connector head portion and guiding entrance of the connector head portion when the connector head portion is inserted into the insertion hole.

An inner wall of the insertion hole may have a polygonal shape, and a polygonal side wall portion having a polygonal shape is provided on an outer wall of the connector head portion to fit to a shape of the inner wall of the insertion hole.

The entrance guide portion is connected to the polygonal side wall portion and disposed below the polygonal side wall portion.

The entrance guide portion includes a guide groove that is formed on an outer wall of the connector head portion.

The guide groove is connected to two side wall edges that are arranged to neighbor each other among a plurality of side wall edges provided on the polygonal side wall portion.

The guide groove is provided in an inclined shape such that a height gradually increases from one side wall edge toward the other side wall edge disposed neighboring to the one side wall edge.

The guide groove may include a plurality of guide grooves that are arranged spaced apart from each other.

The entrance guide portion may further include an initial entrance guiding tip head portion that is provided in the connector head portion, connected to the entrance guide portion, and guides initial entrance into the insertion hole of the connector head portion.

The initial entrance guiding tip head portion may be disposed below the guide groove and may have a convex shape such that an outer diameter of a section thereof decreases in a direction away from the guide groove.

The dental fixture operation connector may further include an elastic pressing body that is provided in the connector head portion, is inserted into an accommodation groove disposed above the entrance guide portion, and elastically presses an inner wall of the insertion hole.

### ADVANTAGEOUS EFFECTS

According to the inventive concept, when the connector head portion is inserted into the insertion hole provided in the fixture, as the entrance guide portion for guiding the entrance of the connector head portion is provided, the connector head portion may be fast and conveniently inserted into the insertion hole, and accordingly the fixture accommodated in the fixture storing ample may be easily picked up out of the ample.

### DESCRIPTION OF THE DRAWINGS

- FIG. 1: schematically illustrates a placement process of a fixture of a dental implant using a dental fixture operation connector according to a first embodiment of the present inventive concept.
- FIG. 2: illustrates the fixture of the dental implant of FIG. 1.
- FIG. 3: is a front side view of FIG. 2.
- FIG. 4: illustrates a section taken along a line A-A of FIG. 3.
- FIG. 5: illustrates the inside of the fixture of FIG. 3.
- FIG. 6: illustrates a dental fixture operation connector that picks up the fixture of FIG. 2.
- FIG. 7: illustrates a connector head portion of FIG. 6.
- FIG. 8: is a bottom view of FIG. 6.
- FIG. 9: illustrates an elastic pressing body that is inserted into an accommodation groove of FIG. 7.
- FIG. 10: is an operation state view illustrating a state in which the connector head portion of FIG. 7 is inserted into the fixture.
- FIG. 11: illustrates a dental fixture operation connector according to the second embodiment of the present inventive concept.

### MODE OF THE INVENTIVE CONCEPT

The attached drawings for illustrating exemplary embodiments of the inventive concept are referred to in order to gain a sufficient understanding of the inventive concept and the merits thereof.

Hereinafter, the inventive concept will be described in detail by explaining exemplary embodiments of the inventive concept with reference to the attached drawings. In the following description, when detailed descriptions about related well-known functions or structures are determined to make the gist of the disclosure unclear, the detailed descriptions will be omitted herein.

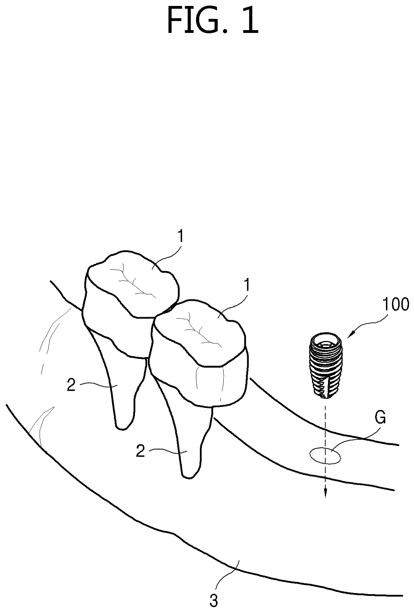

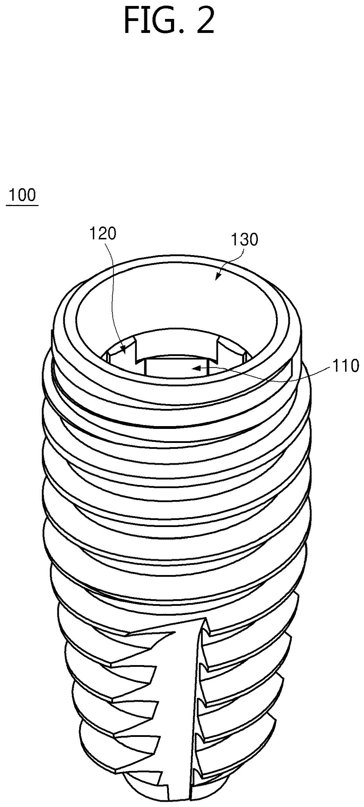

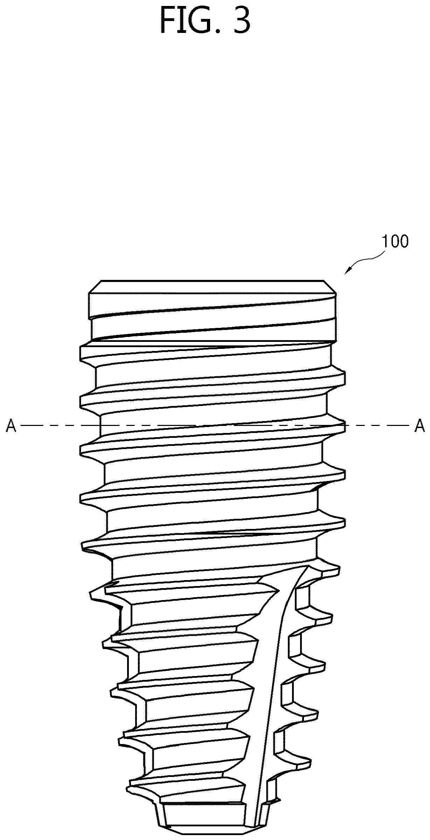

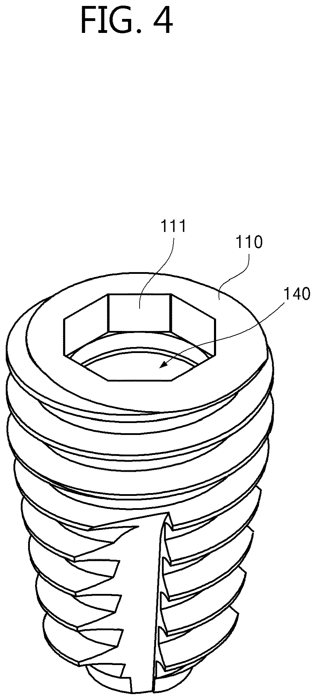

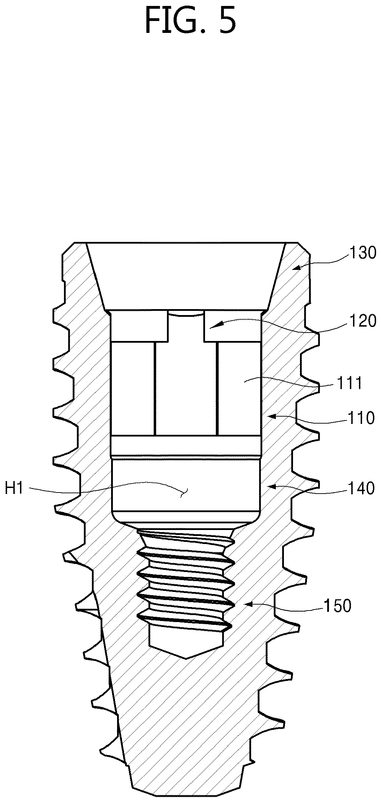

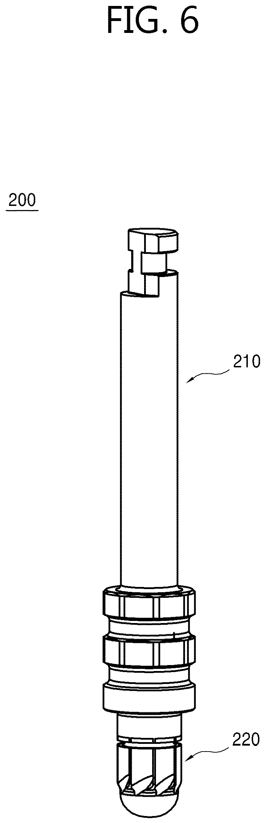

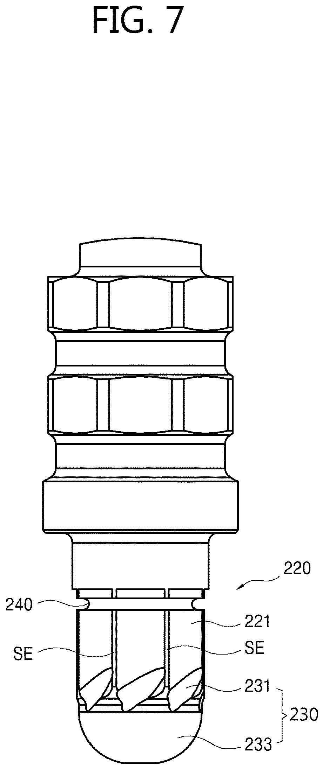

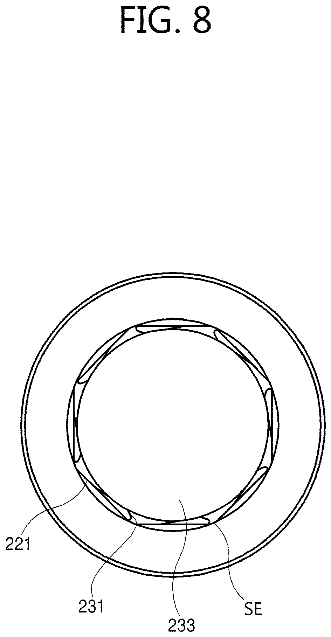

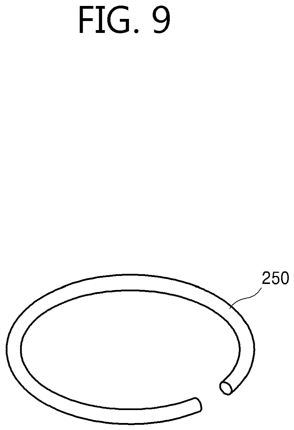

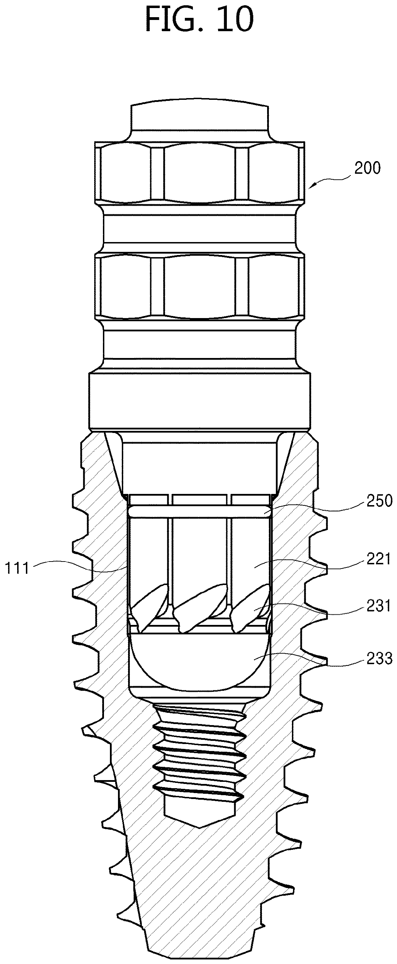

FIG. 1 schematically illustrates a placement process of a fixture of a dental implant using a dental fixture operation connector of the present inventive concept. FIG. 2 illustrates the fixture of the dental implant of FIG. 1. FIG. 3 is a front side view of FIG. 2. FIG. 4 illustrates a section taken along a line A-A of FIG. 3. FIG. 5 illustrates the inside of the fixture of FIG. 3. FIG. 6 illustrates a dental fixture operation connector that picks up the fixture of FIG. 2. FIG. 7 illustrates a connector head portion of FIG. 6. FIG. 8 is a bottom view of FIG. 6. FIG. 9 illustrates an elastic pressing body that is inserted into an accommodation groove of FIG. 7. FIG. 10 is an operation state view illustrating a state in which the connector head portion of FIG. 7 is inserted into the fixture.

As illustrated in FIG. 1, a large number of teeth 1 are arranged in a gum 3. The teeth 1 are primary digestive system that crushes food into pieces and sends them to stomach, and normally include about 28 teeth which may vary person to person.

When any one of the teeth 1 is lost (the posterior is lost), the lost one of the teeth 1 may deteriorate aesthetics and considerably inconvenience chewing food.

Accordingly, a fixture 100 is placed in the gum 3 of the lost one of the teeth 1 as a means for replacing a tooth root 2 of the teeth 1. Although it is not illustrated in detail in FIG. 1, the fixture 100 is placed in an alveolar bone (not shown) in the gum 3, and a drill work for placement of the fixture 100 is performed before the placement. In other words, a drill hole G is processed at a certain position of the alveolar bone.

The fixture 100 may be manufactured of titanium Ti or a Ti alloy that has no rejection to the human body.

For reference, the fixture 100 of the present embodiment that is to be described below may be one used during the initial implant operation or an emergent one used for failure of an operation, which may be directly placed in an alveolar bone (not shown) without supplement of a certain bone substitute material to a damaged area.

An abutment insertion hole H1 (see FIG. 5) into which an abutment (not shown) is inserted is provided in an upper end portion of the fixture 100 by being recessed in the lengthwise direction.

In detail, in the structure of the fixture 100 according to the present embodiment, the fixture 100 according to the present embodiment, as illustrated in FIGS. 2 to 10, may include an abutment rotation prevention portion 110 that prevents a relative rotation with respect to the fixture 100 of the abutment, a tool coupling portion 120 disposed adjacent to the abutment rotation prevention portion 110 in an upper area of the abutment rotation prevention portion 110, a taper portion 130 disposed in an upper area of the tool coupling portion 120, an abutment support portion 140 disposed in a lower area of the abutment rotation prevention portion 110, and a screw fastening portion 150 disposed in the lower area of the abutment support portion 140.

The abutment rotation prevention portion 110 is provided in a shape such that an inner wall 111 that forms the abutment insertion hole H1 has a shape to fit to an outer wall of the abutment. The abutment rotation prevention portion 110 prevents the abutment inserted into the abutment insertion hole H1 from rotation with respect to the fixture 100.

The inner wall 111 of the abutment rotation prevention portion 110 is provided in a polygonal shape along a circumferential direction of the fixture 100. In the present embodiment, the inner wall 111 of the abutment rotation prevention portion 110, as illustrated in detail in FIG. 4, is provided in an octagonal shape along the circumferential direction of the fixture 100.

As the inner wall 111 of the abutment rotation prevention portion 110 is provided in an octagonal shape, the length of one side forming an octagon is less than the length of one side in a conventional hexagonal shape, and accordingly a space between the inner wall 111 of the abutment rotation prevention portion 110 and the outer wall of the abutment is further reduced, thereby preventing the shaking of the abutment coupled to the fixture 100.

The tool coupling portion 120 is connected to the abutment rotation prevention portion 110 and is disposed adjacent to the abutment rotation prevention portion 110 in the upper area of the abutment rotation prevention portion 110.

Meanwhile, the above-described fixture 100 is kept by being stored in a storing ample before an implant operation. During the implant operation, the fixture 100 stored in the storing ample is picked out of the storing ample by using a dental fixture operation connector 200 according to the present embodiment.

The dental fixture operation connector 200 according to the present invention includes as illustrated in FIGS. 6 to 10, a connector body portion 210, a connector head portion 220 connected to the connector body portion 210 and to insert into the abutment insertion hole H1 formed in an upper end area of the fixture 100, an entrance guide portion 230 provided in the connector head portion 220 and guiding entrance of the connector head portion 220 when the connector head portion 220 is inserted into the abutment insertion hole H1. The dental fixture operation connector 200 may include an elastic pressing body 250 provided in the connector head portion 220 and inserted into an accommodation groove 240 provided in an upper portion of the entrance guide portion 230 to elastically press an inner wall of the abutment insertion hole H1.

The connector body portion 210 according to the present embodiment, as illustrated in FIG. 6, is provided in a shape of a bar for chucking having a long length. A handpiece (not shown) may be connected to the connector body portion 210 of the present embodiment. The handpiece supplies a rotational force to the dental fixture operation connector 200 so that the fixture 100 picked up by the dental fixture operation connector 200 may be placed in an alveolar bone (not shown) of a patient.

The connector head portion 220 is connected to the connector body portion 210. The connector head portion 220 is inserted into the abutment insertion hole H1 formed in the upper end area of the fixture 100.

In the present embodiment, a polygonal side wall portion 221 is provided on an outer wall of the connector head portion 220 to be octagonal to fit to the shape of the inner wall 111 of the abutment rotation prevention portion 110 that forms the inner wall of the abutment insertion hole H1.

The polygonal side wall portion 221 closely contact the inner wall 111 of the abutment rotation prevention portion 110 to prevent the fixture 100 from being detached from the dental fixture operation connector 200 in the pick-up process of the fixture 100.

As described above, as the inner wall 111 of the abutment rotation prevention portion 110 and the polygonal side wall portion 221 of the connector head portion 220 are provided in an octagonal shape, when the connector head portion 220 is inserted into the abutment insertion hole H1, if the direction is not accurate, the insertion of the connector head portion 220 is not easy so that use of the dental fixture operation connector 200 may be inconvenient.

To remove inconvenience of the use, the dental fixture operation connector 200 according to the present invention is provided with the entrance guide portion 230 that enables the connector head portion 220 to be easily inserted into the abutment insertion hole H1 even when the direction is not accurate during the insertion of the connector head portion 220 into the abutment insertion hole H1.

The entrance guide portion 230 is provided in the connector head portion 220. When the connector head portion 220 is inserted into the abutment insertion hole H1, the entrance guide portion 230 guides the entrance of the connector head portion 220 so that the polygonal side wall portion 221 of the connector head portion 220 fits to the shape of the inner wall 111 of the abutment rotation prevention portion 110.

To this end, in the present invention, the entrance guide portion 230, as illustrated in detail in FIGS. 7 and 8, is connected to the polygonal side wall portion 221 and disposed below the polygonal side wall portion 221.

As such, as the entrance guide portion 230 according to the present invention is connected to the polygonal side wall portion 221 and disposed below the polygonal side wall portion 221, when the connector head portion 220 is inserted into the abutment insertion hole H1, the initial entrance of the connector head portion 220 may be guided such that the polygonal side wall portion 221 of the connector head portion 220 fits to the shape of the inner wall 111 of the abutment rotation prevention portion 110.

According to the present invention, the entrance guide portion 230, as illustrated in detail in FIGS. 6 to 10, includes a guide groove 231 formed on the outer wall of the connector head portion 220. Said guide groove 231 being recessed in a direction along the virtual center axis line of the connector head portion 220, and an initial entrance guiding tip head portion 233 provided in the connector head portion 220, connected to the entrance guide portion 230, and guiding initial entrance of the connector head portion 220 into.

The guide groove 231 is recessed in the outer wall of the connector head portion 220 in the direction along the virtual center axis line of the connector head portion 220. The guide groove 231, as illustrated in detail in FIGS. 7 and 8, is connected to two side wall edges (SE) arranged neighboring each other among the eight side wall edges SE provided in the polygonal side wall portion 221.

In the present embodiment, the guide groove 231 is provided to be connected to the two side wall edges SE arranged neighboring each other among the eight side wall edges SE provided in the polygonal side wall portion 221.

Furthermore, the guide groove 231 according to the present invention, as illustrated in detail in FIGS. 7 and 8, has a shape inclined to have a height gradually increasing from a side wall edge SE at one side to another side wall edge SE at the other side disposed neighboring the wall edge SE at one side.

As such, as the guide groove 231 in an oblique direction and connects the neighboring side wall edges SE of the polygonal side wall portion 221, when the connector head portion 220 is inserted into the abutment insertion hole H1, even if the directions of the polygonal side wall portion 221 of the connector head portion 220 and the inner wall 111 of the abutment rotation prevention portion 110 do not match accurately, the dental fixture operation connector 200 or the fixture 100 may be rotated along the guide groove 231 disposed in the oblique direction, and accordingly the connector head portion 220 is easily inserted into the abutment insertion hole H1 so that the polygonal side wall portion 221 fits to the shape of the inner wall 111 of the abutment rotation prevention portion 110.

In the present embodiment, the guide groove 231, as illustrated in detail in FIGS. 7 and 8, may include eight guide grooves arranged spaced apart from each other. The guide grooves 231 are radially arranged with respect to the virtual center axis line of the connector head portion 220.

As such, as the guide grooves 231 are radially arranged with respect to the virtual center axis line of the connector head portion 220, when the connector head portion 220 is inserted into the abutment insertion hole H1, the entrance of the connector head portion 220 may be stably guided.

The initial entrance guiding tip head portion 233 is provided in the connector head portion 220. The initial entrance guiding tip head portion 233 is connected to the entrance guide portion 230 to guide the initial entrance toward the abutment insertion hole H1.

The initial entrance guiding tip head portion 233 according to the present embodiment, as illustrated in detail in FIG. 7, is disposed below the guide groove 231. Furthermore, the initial entrance guiding tip head portion 233 according to the present embodiment has a convex hemispherical shape such that the outer diameter of a section thereof decreases in a direction away from the guide groove 231.

As such, as the initial entrance guiding tip head portion 233 has a convex hemispherical shape, during the initial entrance of the connector head portion 220 into the abutment insertion hole H1, regardless of the direction of the connector head portion 220, the connector head portion 220 may smoothly enters the abutment insertion hole H1.

Meanwhile, in the connector head portion 220, as illustrated in detail in FIG. 7, the accommodation groove 240 is disposed above the entrance guide portion 230. The elastic pressing body 250, which elastically presses the inner wall of the abutment insertion hole H1, is inserted into the accommodation groove 240.

In the present embodiment, the accommodation groove 240 is formed in the upper area of the entrance guide portion 230 and the polygonal side wall portion 221.

As illustrated in detail in FIG. 9, the elastic pressing body 250 is provided in a ring shape having a partially cut portion.

The elastic pressing body 250 elastically presses the inner wall 111 of the abutment rotation prevention portion 110 while the connector head portion 220 is inserted into the abutment insertion hole H1. As such, as the connector head portion 220 according to the present embodiment elastically presses the inner wall 111 of the abutment rotation prevention portion 110, the fixture 100 may be prevented from being detached from the dental fixture operation connector 200 in the pick-up process of the fixture 100 from the storing ample.

Furthermore, in the present embodiment, as the elastic pressing body 250 is disposed in the upper area of the entrance guide portion 230 and the polygonal side wall portion 221, in the insertion process of the connector head portion 220, to a certain insertion depth, the elastic pressing body 250 does not contact the inner wall 111 of the abutment rotation prevention portion 110, causing no insertion resistance applied by the elastic pressing body 250, and thus the initial insertion of the connector head portion 220 is facilitated.

Furthermore, the dental fixture operation connector 200 according to the present embodiment may place the fixture 100 in the alveolar bone of a patient through rotation while the fixture 100 is coupled thereof.

The operation of the dental fixture operation connector 200 of the present embodiment is described below with reference to FIGS. 1 to 10.

To pick up the fixture 100 stored in the storing ample from the storing ample, the dental fixture operation connector 200 of the present embodiment is moved over the fixture 100.

As the dental fixture operation connector 200 that is moved over the fixture 100 descends, the initial entrance guiding tip head portion 233 first enters the inside of the abutment insertion hole H1.

As described above, as the initial entrance guiding tip head portion 233 is provided in a hemispherical shape, the connector head portion 220 may smoothly and initially enter abutment insertion hole H1.

Next, as the dental fixture operation connector 200 continuously descends, the guide groove 231 contacts the inner wall 111 of the abutment rotation prevention portion 110. The guide groove 231 according to the present invention is disposed in an oblique direction to connect the two neighboring side wall edges SE of the polygonal side wall portion 221, and thus even when the directions of the polygonal side wall portion 221 of the connector head portion 220 and the inner wall 111 of the abutment rotation prevention portion 110 do not match accurately, the dental fixture operation connector 200 or the fixture 100 may be rotated along the guide groove 231 disposed in the oblique direction. Accordingly, the connector head portion 220 is easily inserted into the abutment insertion hole H1, and thus the polygonal side wall portion 221 fits to the shape of the inner wall 111 of the abutment rotation prevention portion 110.

In this state, the elastic pressing body 250 contacts the inner wall 111 of the abutment rotation prevention portion 110 to elastically press the inner wall 111 of the abutment rotation prevention portion 110.

Next, the dental fixture operation connector 200 ascends and picks up the fixture 100 from the storing ample. In this state, as the polygonal side wall portion 221 fits to the shape of the inner wall 111 of the abutment rotation prevention portion 110, the polygonal side wall portion 221 and the inner wall 111 of the abutment rotation prevention portion 110 are in close contact with each other, and as the elastic pressing body 250 presses the inner wall 111 of the abutment rotation prevention portion 110, the fixture 100 is not detached from the dental fixture operation connector 200 in the pick-up process of the fixture 100.

Thereafter, the fixture 100 that is picked up by the dental fixture operation connector 200 is moved into the mouth of a patient, and due to the rotation of the dental fixture operation connector 200, the fixture 100 is placed in the alveolar bone of a patient.

As such, as the dental fixture operation connector 200 according to the present invention is provided the entrance guide portion 230 for guiding the entrance of the connector head portion 220 when the connector head portion 220 is inserted into the abutment insertion hole H1 provided in the fixture 100, the connector head portion 220 may be fast and conveniently inserted into the abutment insertion hole H1, and thus the fixture 100 stored in the storing ample may be easily picked up out of the ample.

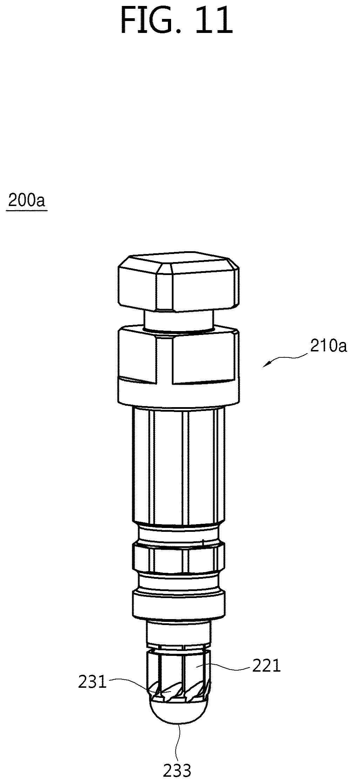

FIG. 11 illustrates a dental fixture operation connector according to the second embodiment of the present inventive concept.

Compared to the first embodiment, the present embodiment differs in the shape of the connector body portion 210 only, but the other features are the same as those of the first embodiment in FIGS. 1 to 10. Accordingly, in the following description, like reference numerals are used for like elements and descriptions thereof are omitted.

As illustrated in FIG. 11, a connector body portion 210a of a dental fixture operation connector 200a according to the present embodiment is provided in a square block shape having a shorter length and a wider width than that of the first embodiment.

As such, as the connector body portion 210a according to the present embodiment is provided in a square block shape having a wide width, an operator may easily grip and handle the connector.

Although the present embodiment is described in detail with reference to the accompanying drawings, the scope of rights of the present embodiment is not limited to the above-mentioned drawings and descriptions.

As such, it is obvious to one of skilled in the art that the present inventive concept is not limited to the above-described embodiment and but that the invention is defined by the scope of the following claims.

### Industrial Applicability

The present inventive concept may be used for a medical industry, particularly for a dental medical industry.

## Claims

1. A dental fixture operation connector (200) including a connector body portion (210), a connector head portion (220) connected to the connector body portion (210) and configured to be inserted into an insertion hole (H1) formed in an upper end area of a fixture (100), and an entrance guide portion (230) provided on the connector head portion (220) and guiding entrance of the connector head portion (220) when the connector head portion (220) is inserted into the insertion hole (H1), wherein a polygonal side wall portion (221) having a polygonal shape is provided on an outer wall of the connector head portion (220) to fit to a polygonal shape of the inner wall (11) of the insertion hole (H1), wherein the entrance guide portion (230) is connected to the polygonal side wall portion (221) and disposed below the polygonal side wall portion (221), wherein the entrance guide portion (230) includes a guide groove (231) that is formed on the outer wall of the connector head portion (220), wherein the guide groove (231) is connected to two side wall edges (SE) that are arranged to neighbor each other among a plurality of side wall edges (SE) provided on the polygonal side wall portion (221), wherein the guide groove (231) has a shape inclined to have a height gradually increasing from one side wall edge (SE) toward the other side wall edge (SE) disposed neighboring to the one side wall edge (SE).

2. The dental fixture operation connector (200) of claim 1, wherein the guide groove (231) includes a plurality of guide grooves (231) that are arranged spaced apart from each other.

3. The dental fixture operation connector (200) of claim 1, wherein the entrance guide portion (230) further includes an initial entrance guiding tip head portion (233) that is provided in the connector head portion (220), connected to the entrance guide portion (230), and guides initial entrance into the insertion hole (H1).

4. The dental fixture operation connector (200) of claim 3, wherein the initial entrance guiding tip head portion (233) is disposed below the guide groove (231) and has a convex shape such that an outer diameter of a section thereof decreases in a direction away from the guide groove (231).

5. The dental fixture operation connector (200) of claim 1, further including an elastic pressing body (250) that is provided in the connector head portion (220), is inserted into an accommodation groove (240) disposed above the entrance guide portion (230), and elastically presses an inner wall (111) of the insertion hole (H1).

## Patentansprüche

1. Chirurgischer Verbinder für Dentalvorrichtung (200), einschließend einen Verbinderkörperanteil (210), einen Verbinderkopfanteil (220), der mit dem Verbinderkörperanteil (210) verbunden ist und ausgestaltet ist, um in ein Einsetzloch (H1) eingesetzt zu werden, das in einem oberen Endbereich einer Vorrichtung (100) gebildet ist, und einen Eingangsführungsanteil (230), der auf dem Verbinderkopfanteil (220) bereitgestellt ist und den Eingang des Verbinderkopfanteils (220) führt, wenn der Verbinderkopfanteil (220) in das Einsetzloch (H1) eingesetzt wird, wobei
ein vieleckiger Seitenwandanteil (221) mit einer vieleckigen Form auf einer Außenwand des Verbinderkopfanteils (220) bereitgestellt wird, um zu einer vieleckigen Form der Innenwand (11) des Einsetzlochs (H1) zu passen, wenn der Eingangsführungsanteil (230) mit dem vieleckigen Seitenwandanteil (221) verbunden wird und unter dem vieleckigen Seitenwandanteil (221) angeordnet wird, wobei der Eingangsführungsanteil (230) eine Führungsrille (231) einschließt, die auf der Außenwand des Verbinderkopfanteils (220) gebildet ist, wobei die Führungsrille (231) an zwei Seitenwandrändern (SE) verbunden ist, die so vorgesehen sind, dass sie innerhalb einer Vielzahl von Seitenwandrändern (SE), die auf dem vieleckigen Seitenwandanteil (221) bereitgestellt werden, einander benachbart sind, wobei die Führungsrille (231) eine schräge Form hat, um eine Höhe zu haben, die von einem Seitenwandrand (SE) in Richtung des anderen Seitenwandrandes (SE), der benachbart zu dem einen Seitenwandrand (SE) angeordnet ist, allmählich zunimmt.

2. Chirurgischer Verbinder für Dentalvorrichtung (200) nach Anspruch 1, wobei die Führungsrille (231) eine Vielzahl von Führungsrillen (231) einschließt, die voneinander beabstandet vorgesehen sind.

3. Chirurgischer Verbinder für Dentalvorrichtung (200) nach Anspruch 1, wobei der Eingangsführungsanteil (230) des Weiteren eine anfängliche Eingangsführungsspitze des Kopfanteils (233) einschließt, die in dem Verbinderkopfanteil (220) bereitgestellt ist, mit dem Eingangsführungsanteil (230) verbunden ist und den anfänglichen Eingang in das Einsetzloch (H1) führt.

4. Chirurgischer Verbinder für Dentalvorrichtung (200) nach Anspruch 3, wobei die anfängliche Eingangsführungsspitze des Kopfanteils (233) unter der Führungsrille (231) angeordnet ist und eine konvexe Form hat, so dass ein Außendurchmesser eines Segments davon in einer Richtung von der Führungsrille (231) weg abnimmt.

5. Chirurgischer Verbinder für Dentalvorrichtung (200) nach Anspruch 1, des Weiteren einschließend einen elastischen Andruckkörper (250), der in dem Verbinderkopfanteil (220) bereitgestellt wird, in eine Aufnahmenut (240) eingesetzt wird, die oberhalb des Eingangsführungsanteils (230) angeordnet ist, und eine Innenwand (111) des Einsetzlochs (H1) elastisch andrückt.

## Revendications

1. Connecteur opérationnel de fixation dentaire (200) incluant une partie de corps de connecteur (210), une partie de tête de connecteur (220) connectée à la partie de corps de connecteur (210) et configurée pour être insérée dans un trou d'insertion (H1) formé dans une zone d'extrémité supérieure d'une fixation (100), et une partie de guidage d'entrée (230) prévue sur la partie de tête de connecteur (220) et guidant l'entrée de la partie de tête de connecteur (220) lorsque la partie de tête de connecteur (220) est insérée dans le trou d'insertion (H1), dans lequel
une partie de paroi latérale polygonale (221) ayant une forme polygonale est prévue sur une paroi extérieure de la partie de tête de connecteur (220) pour s'adapter à une forme polygonale de la paroi intérieure (11) du trou d'insertion (H1), dans lequel la partie de guidage d'entrée (230) est connectée à la partie de paroi latérale polygonale (221) et disposée sous la partie de paroi latérale polygonale (221), dans lequel la partie de guidage d'entrée (230) inclut une rainure de guidage (231) qui est formée sur la paroi extérieure de la partie de tête de connecteur (220), dans lequel la rainure de guidage (231) est connectée à deux bords de paroi latérale (SE) qui sont agencés pour être voisins l'un de l'autre parmi une pluralité de bords de paroi latérale (SE) prévus sur la partie de paroi latérale polygonale (221), dans lequel la rainure de guidage (231) présente une forme inclinée pour avoir une hauteur augmentant graduellement d'un bord de paroi latérale (SE) vers l'autre bord de paroi latérale (SE) disposé voisin de l'un bord de paroi latérale (SE).

2. Connecteur opérationnel de fixation dentaire (200) selon la revendication 1, dans lequel la rainure de guidage (231) inclut une pluralité de rainures de guidage (231) qui sont agencées espacées l'une de l'autre.

3. Connecteur opérationnel de fixation dentaire (200) selon la revendication 1, dans lequel la partie de guidage d'entrée (230) inclut en outre une partie de tête d'embout de guidage d'entrée initiale (233) qui est prévue dans la partie de tête de connecteur (220), connectée à la partie de guidage d'entrée (230), et guide l'entrée initiale dans le trou d'insertion (H1).

4. Connecteur opérationnel de fixation dentaire (200) selon la revendication 3, dans lequel la partie de tête d'embout de guidage d'entrée initiale (233) est disposée sous la rainure de guidage (231) et a une forme convexe telle qu'un diamètre extérieur d'une section de celle-ci décroît dans une direction s'éloignant de la rainure de guidage (231).

5. Connecteur opérationnel de fixation dentaire (200) selon la revendication 1, incluant en outre un corps de compression élastique (250) qui est prévu dans la partie de tête de connecteur (220), est inséré dans une rainure de logement (240) disposée au-dessus de la partie de guidage d'entrée (230) et comprime élastiquement une paroi intérieure (111) du trou d'insertion (H1).
